# EUROPEAN PATENT APPLICATION

(11) **EP 4 144 326 A1**
(43) Date of publication of application: **08.03.2023**
(21) Application number: 22192960.7
(22) Date of filing: 30.08.2022
(51) Int. Cl.: A61F 2/07

(54) **ENCAPSULATED DEVICES WITH SEPARATION LAYERS**

(30) Priority: 03.09.2021 US 202163240424 P
(71) Applicant: Cook Medical Technologies LLC, Bloomington, IN 47404 (US)
(72) Inventor: MAJERCAK, David, Indiana, 47401 (US)
(74) Representative: Williams Powell

(57) **Abstract**

Disclosed herein is an encapsulated device comprising at least one stent that allows for improved flexibility and tailoring to specific needs by a user by selection of features along the lengths and/or circumferences of the encapsulated devices, and that provides enhanced radio-opacity at predetermined locations of the encapsulated device.

## Description

### FIELD OF THE INVENTION

The present disclosure relates generally to medical devices and methods for treating medical conditions, and more specifically, for encapsulated devices including stents and stent-grafts incorporating separation layers for use in body vessels to treat those medical conditions.

### BACKGROUND OF THE INVENTION

Covered stents have come into accepted use for preferred treatment in such applications as bridging stents for aortic stent-grafts and iliac occlusive disease, where the polymeric covering adds to the effective long-term performance of the covered stents.

For the application as a bridging stent for fenestrated and branched stent-grafts, covered stents are used almost exclusively, despite no current covered stent having yet been approved for the bridging indication.

Generally, covered balloon-expandable stents have been preferred over sheathed self-expanding covered stents in fenestrations due to covered balloon-expandable stents having observed advantages, including a lower delivery profile, a higher radial strength, and an improved placement accuracy. However, for branches, primarily due to the observed lack of expanded flexibility, self-expanding covered stents have been preferred.

Thus, there remains a need for further contributions in this area of technology.

### SUMMARY OF THE INVENTION

The present invention seeks to provide an improved medical device, in particular an encapsulated device comprising a stent.

According to an aspect of the present invention, an encapsulated device is provided. The encapsulated device includes at least one stent, a biocompatible covering encapsulating the at least one stent. The biocompatible covering includes a generally tubular body, a proximal end, a distal end, a central region disposed between the proximal end and the distal end, a lumen extending between the proximal end and the distal end, a first layer, a second layer radially outward from the first layer, and a third layer radially outward from the second layer. The encapsulated device also includes a first separation layer encapsulating at least the central region of the biocompatible covering disposed between the first layer and the second layer of the biocompatible covering. The encapsulated device also includes a second separation layer encapsulating at least the central region of the biocompatible covering disposed between the second layer and the third layer of the biocompatible covering. The biocompatible covering encapsulates the at least one stent internal and/or external to the at least one stent, the at least one stent disposed along a length of the biocompatible covering.

According to another aspect of the present invention, an encapsulated device is provided. The encapsulated device includes at least one stent, a biocompatible covering encapsulating the at least one stent. The biocompatible covering includes a generally tubular body, a proximal end, a distal end, a central region disposed between the proximal end and the distal end, a lumen extending between the proximal end and the distal end, a first layer, a second layer radially outward from the first layer, a third layer radially outward from the second layer, and at least two circumferential zones in an axial direction between the proximal end and the distal end. The encapsulated device also includes a first separation layer encapsulating each of the at least two circumferential zones, the first separation layer disposed between the first layer and the second layer of the biocompatible covering. The encapsulated device also includes a second separation layer encapsulating each of the at least two circumferential zones, the second separation layer disposed between the second layer and the third layer of the biocompatible covering. The biocompatible covering encapsulates the at least one stent internal and/or external to the at least one stent, the at least one stent disposed along a length of the biocompatible covering proximal or distal to each of the at least two circumferential zones.

According to another aspect of the present invention, an encapsulated device is provided. The encapsulated device includes at least one stent, a biocompatible covering encapsulating the at least one stent. The biocompatible covering includes a generally tubular body, a proximal end, a distal end, a central region disposed between the proximal end and the distal end, a lumen extending between the proximal end and the distal end, a first layer, a second layer radially outward from the first layer, and a third layer radially outward from the second layer. The encapsulated device also includes a first separation layer encapsulating at least the central region of the biocompatible covering disposed between the first layer and the second layer of the biocompatible covering. The encapsulated device also includes a second separation layer encapsulating at least the central region of the biocompatible covering disposed between the second layer and the third layer of the biocompatible covering. The biocompatible covering encapsulates the at least one stent internal and/or external to the at least one stent, the at least one stent disposed along a length of the biocompatible covering. The first layer is oriented in a circumferential direction about the at least one stent. The second layer is oriented in a second circumferential direction opposite the circumferential direction. The third layer is oriented in the circumferential direction.

Further aspects, advantages and areas of applicability will become apparent from the specific description that follows. It should be understood that the description and specific embodiments are intended for purposes of illustration only and are not intended to limit the scope of the claims.

### BRIEF DESCRIPTION OF THE DRAWINGS

Embodiments of the present invention are described below, with reference to the accompanying drawings. The components in the drawings are not necessarily to scale.
FIG. **1** illustrates a perspective view of the components of an assembly of an embodiment of an encapsulated device according to the principles of the present disclosure;
FIG. **2** illustrates a perspective view of the components of an assembly of another embodiment of an encapsulated device according to the principles of the present disclosure;
FIG. **3** illustrates a perspective view of the components of an assembly of another embodiment of an encapsulated device according to the principles of the present disclosure;
FIG. **4** illustrates an end view of another embodiment of an encapsulated device assembled according to the principles of the present disclosure;
FIG. **4A** illustrates an exploded partial end view of the embodiment of an encapsulated device assembled according to the principles of the present disclosure of FIG. **4**;
FIG. **5** illustrates a perspective view of the components of an assembly of another embodiment of an encapsulated device according to the principles of the present disclosure;
FIG. **6** illustrates an end view of the encapsulated device of the embodiment of an encapsulated device assembled according to the principles of the present disclosure of FIG. **5**;
FIG. **6A** illustrates an exploded partial end view of the embodiment of an encapsulated device assembled according to the principles of the present disclosure of FIG. 6; and
FIG. **7** illustrates a perspective view of another embodiment of an encapsulated device according to the principles of the present disclosure.

The drawings described herein are for illustration purposes only and are not intended to limit the scope of the present disclosure in any way.

### DESCRIPTION OF THE PREFERRED EMBODIMENTS

in adding reference denotations to elements of each drawing, although the same elements are displayed on a different drawing, it should be noted that the same elements have the same denotations. In addition, in describing one aspect of the present disclosure, where it has been deemed that a detailed description of related well-known configurations or functions is unnecessary, it has been omitted.

In the following discussion, the terms "proximal" and "distal" are used to describe the opposing axial ends of the device, as well as the axial ends of various component features. The term "proximal" is used in its conventional sense to refer to the end of the device (or component) that is closest to the medical professional during use of the assembly. The term "distal" is used in its conventional sense to refer to the end of the device (or component) that is initially inserted into the patient, or that is closest to the patient during use. The term "longitudinal" is used to refer to an axis that aligns with the proximal-distal axis of the device (or component). The terms "radially" and "radial" are used to refer to elements, surfaces, or assemblies relative to one another that may extend perpendicularly or substantially perpendicularly from a longitudinal axis. The terms "external" and "radially outward," and "internal" and "radially inward," are used to refer to elements, surfaces, or assemblies relative to one another extending perpendicularly from a longitudinal axis, "external" or "radially outward" referring, in context, to elements, surfaces, or assemblies relatively further along a radius from a longitudinal axis than elements, surfaces, or assemblies referred to as "internal" or "radially inward" to such an "external" or "radially outward" element, surface, or assembly. The terms "circumference," "circumferentially," and "circumferential" are used to refer to elements, surfaces, or assemblies relative to one another encircling a longitudinal axis at a radius.

The uses of the terms "a" and "an" and "the" and similar references in the context of describing the present disclosure (especially in the context of the following claims) are to be construed to cover both the singular and the plural, unless otherwise indicated herein or clearly contradicted by context. The use of the term "plurality of" is defined by the Applicant in the broadest sense, superseding any other implied definitions or limitations hereinbefore or hereinafter unless expressly asserted by the Applicant to the contrary, to mean a quantity of more than one. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

As used herein the terms "comprise(s)," "include(s)," "having," "has," "can," "may," "contain(s)," and variants thereof, are intended to be open-ended transitional phrases, terms, or words that do not preclude the possibility of additional acts or structures. The present description also contemplates other embodiments "comprising," "consisting of," and "consisting essentially of," the examples or elements presented herein, whether explicitly set forth or not.

In describing elements of the present disclosure, the terms 1^{st}, 2^{nd}, first, second, A, B, (a), (b), and the like may be used herein. These terms are only used to distinguish one element from another element, but do not limit the corresponding elements irrespective of the nature or order of the corresponding elements.

Unless otherwise defined, all terms used herein, including technical or scientific terms, have the same meanings as those generally understood by those skilled in the art to which the present disclosure pertains. Such terms as those defined in a generally used dictionary are to be interpreted as having meanings equal to the contextual meanings in the relevant field of art.

As used herein, the term "about," when used in the context of a numerical value or range set forth means a variation of ±15%, or less, of the numerical value. For example, a value differing by ±15%, ±14%, ±10%, or ±5%, among others, would satisfy the definition of "about," unless more narrowly defined in particular instances.

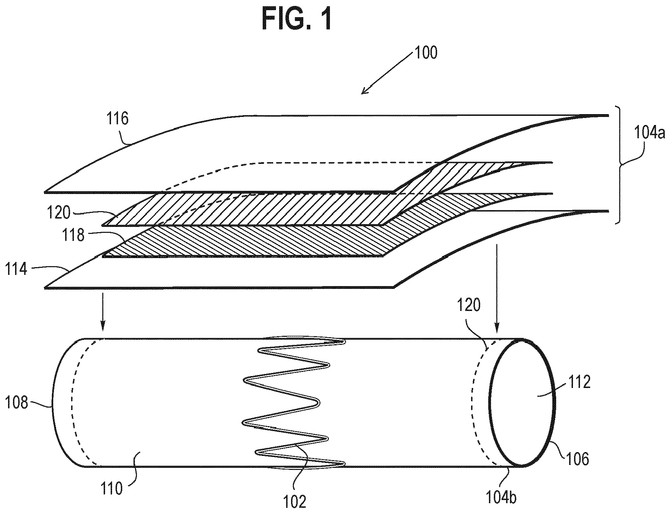

Referring to FIG. **1**, a perspective view of the components of an assembly of an embodiment of an encapsulated device **100** according to the principles taught herein is illustrated. Encapsulated device **100** includes at least one stent **102**. At least one stent **102** is external to and about the circumference of biocompatible covering **104,** which has a generally tubular body. Biocompatible covering **104** extends longitudinally from proximal end **106** to distal end **108** and includes a lumen **112** extending longitudinally from proximal end **106** to distal end **108**. Biocompatible covering **104** may include a first portion **104a,** the components of which are shown in FIG. **1** being applied to the exterior of at least one stent **102**. Biocompatible covering **104** may include a second portion **104b,** which is shown assembled to the interior of at least one stent **102** in FIG. **1**. Biocompatible covering **104,** as illustrated in FIG. **1** by first portion **104a,** includes a first layer **114** and a second layer **116,** second layer **116** external (i.e., radially outward) relative to first layer **114**. Between first layer **114** and second layer **116** are first separation layer **118** and second separation layer **120.** FIG. **1** illustrates first layer **114** and second layer **116** of first portion **104a** of biocompatible covering **104,** with first separation layer **118** and second separation layer **120** together between first layer **114** and second layer **116,** being applied external (i.e., radially outward) relative to at least one stent **102**. Another first layer **114** and second layer **116** of second portion **104b** of biocompatible covering **104,** with first separation layer **118** and second separation layer **120** together between first layer **114** and second layer **116,** is internal (i.e., radially inward) relative to at least one stent **102**. First separation layer **118** and second separation layer **120** encapsulate at least central region **110** of biocompatible covering **104**. Though not shown in FIG. **1****,** first separation layer **118** and second separation layer **120** may extend to proximal end **106** and/or distal end **108** as is preferable or desirable. The lack of adherence and/or bonding of first layers **114** to second layers **116** can contribute to the enhanced flexibility and an added degree of freedom of encapsulated device **100** in that the encapsulated device **100** can move more or less freely with respect to at least one stent **102**. Encapsulated device **100** does not suffer the disadvantages of pin-holing and creep resistance known in the art to occur with devices that are not fully encapsulated.

The at least one stent **102** can be made from a variety of metals and alloys. In one embodiment, at least one stent **102** is made from a shape-memory material such as a nickel-titanium alloy ("nitinol"). Moreover, the structure of at least one stent **102** may be formed in a variety of ways to provide a suitable intraluminal support structure. For example, at least one stent **102** may be made from a woven wire structure, a laser-cut cannula, individual interconnected rings, or another pattern or design.

In another embodiment, as depicted in FIG. **1**, at least one stent **102** may be configured in the form of at least one "Z-stents" or Gianturco^{™} stents, each of which can include a series of substantially straight segments interconnected by a series of bent segments. The bent segments may include acute bends or apices. The Gianturco^{™} stents are arranged in a zigzag configuration in which the straight segments are set at angles relative to each other and are connected by the bent segments. In an embodiment, multiple stents, such as two or more stents, may be discretely spaced-apart in an axial direction from one another between proximal end **106** and distal end **108** of biocompatible covering **104**.

First layers **114** and second layers **116** of biocompatible covering **104** may include a polymeric sheet having any suitable porosity. The porosity may be substantially porous or substantially non-porous and may be selected depending on the application. In one example, a porous polymeric sheet includes the polyurethane Thoralon^{®}. In addition to, or in lieu of, a porous polyurethane, examples of biocompatible covering **104** can include, but are not limited to, biocompatible polymeric materials such as non-porous polyurethanes, polytetrafluoroethylene ("PTFE"), expanded PTFE ("ePTFE"), polyethylene tetraphthalate ("PET"), aliphatic polyoxaesters, polylactides, polycaprolactones, and hydrogels. Biocompatible covering **104** may include Dyneema ultra-high molecular weight polyethylene ("UHMwPE"). Biocompatible covering **104** may include a graft material, such as Dacron^{®}, which may optionally be heat treated and/or partially melted.

Where biocompatible covering **104** includes ePTFE, it is advantageous that at least first layer **114** and second layer **116** of biocompatible covering **104** are composed of ultra-thin ePTFE, because there are substantially different mechanical properties between multiple layers of ePTFE and a single layer of ePTFE. At least one layer of biocompatible covering **104** may be internal to at least one stent **102**, and at least one layer of biocompatible covering **104** may be external to at least one stent **102.** Biocompatible covering **104** may include additional layers as thin as 0.1 micrometres that are sintered together. Advantageously, one or more separation layers such as first separation layer **118** and second separation layer **120** can be inserted between layers of biocompatible covering **104**, which may effectively prevent ePTFE-to-ePTFE bonding.

A separation layer such as first separation layer **118** and second separation layer **120** may include a foil that includes one or more metals. The one or more metals may be radiopaque metals, such that a separation layer is a radiopaque material layer. Examples of radiopaque metals include a metal or one or more metals selected from the group consisting of gold, platinum, palladium, rhodium, titanium, silver, and tungsten. In case of accidental exposure of a separation layer to blood flow, gold has the advantageous characteristic of being anti-thrombogenic, and silver is known to have anti-bacterial, viral, and inflammatory properties. The foil may be such that where first separation layer **118** and second separation layer **120** are disposed between first layer **114** and second layer **116** of biocompatible covering **104**, first layer **114** of biocompatible covering **104** is not adhered to second layer **116**. First separation layer **118** and second separation layer **120** may each be up to, or at least, 25 micrometres thick. Examples of other thickness of first separation layer **118** and second separation layer **120** may include up to, or at least, 50 micrometres, 75 micrometres, 100 micrometres, 125 micrometres, 150 micrometres, 175 micrometres, 200 micrometres, 225 micrometres, 250 micrometres, 500 micrometres, 750 micrometres, 1000 micrometres, 1250 micrometres, 1500 micrometres, 1750 micrometres, 2000 micrometres, 2250 micrometres, or 2500 micrometres. In an example, a single separation layer of 0.15 micrometre foil would contribute 0.1% of cross-sectional area of a 6F sheath compatible device when wrapped in foil at an 8-millimeter diameter. Encapsulated device **100** may include a third separation layer between biocompatible covering **104** and at least one stent **102.**

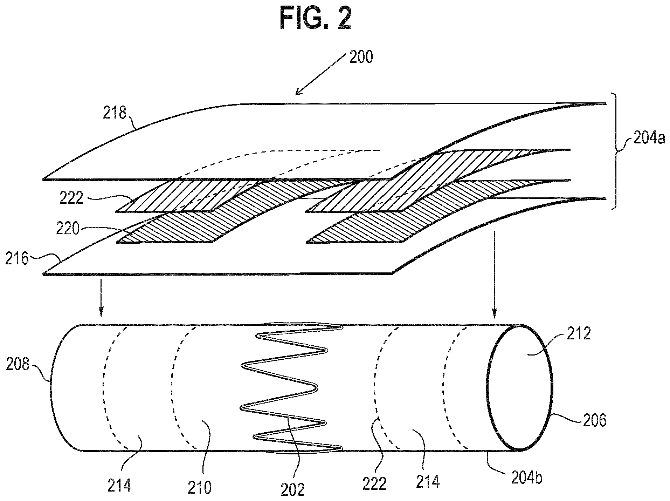

Referring to FIG. **2**, a perspective view of the components of an assembly of another embodiment of an encapsulated device **200** according to the principles of the present disclosure is illustrated. Encapsulated device **200** includes at least one stent **202**. At least one stent **202** is external to and about the circumference of biocompatible covering **204,** which has a generally tubular body. Second portion **204b** of biocompatible covering **204** extends from proximal end **206** to distal end **208** and includes lumen **212** extending longitudinally from proximal end **206** to distal end **208**. As illustrated in FIG. **2****,** first portion **204a** of biocompatible covering **204** illustrates the assembly of first portion **204a,** including a first layer **216** and a second layer **218,** second layer **218** external to first layer **216.** Between first layer **216** and second layer **218** are first separation layers **220** and second separation layers **222.** FIG. **2** illustrates first layer **216** and second layer **218** of biocompatible covering **204**, with at least two discrete spaced-apart first separation layers **220** and second separation layers **222,** each first separation layer **220** together with a second separation layer **222** between first layer **216** and second layer **218,** being applied external relative to at least one stent **202**.

Another first layer **216** and second layer **218** of second portion **204b** of biocompatible covering **204,** with at least two discrete spaced-apart first separation layers **220** and second separation layers **222,** each first separation layer **220** together with a second separation layer **222** between first layer **216** and second layer **218,** is internal relative to at least one stent **202,** illustrated in FIG. **2** as already assembled. The outlines of first separation layers **216** and second separation layers **222** between first layer **216** and second layer **218** are indicated by the at least two circumferential zones **214,** which indicate that the first separation layers **220** and second separation layers **222** span the circumference of encapsulated device **200**. By including at least two discrete spaced-apart first separation layers **220** and second separation layers **222,** each first separation layer **220** together with a second separation layer **222** between first layer **216** and second layer **218,** the thicker or multiple layers of foil achieve zones of enhanced radio-opacity. Additionally, the lack of adherence between first layers **216** and second layers **218** of biocompatible covering **204** contributes to the enhanced flexibility and freedom of movement of biocompatible covering **204** relative to at least one stent **202**.

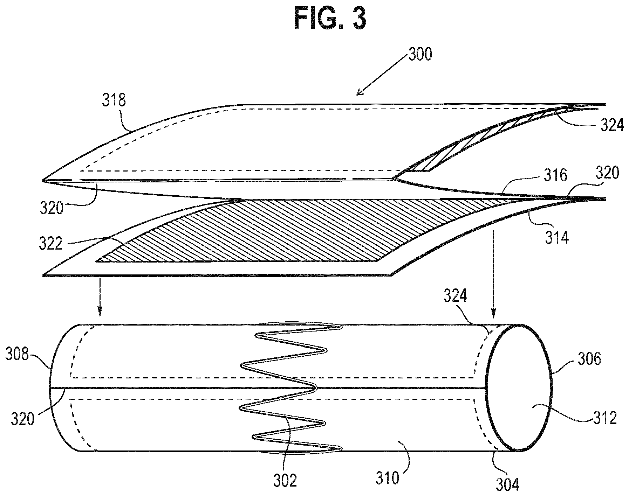

Referring to FIG. **3**, a perspective view of the components of an assembly of another embodiment of an encapsulated device **300** according to the principles of the present disclosure is illustrated. Encapsulated device **300** includes at least one stent **302**. At least one stent **302** is external to and about the circumference of biocompatible covering **304**, which has a generally tubular body. Biocompatible covering **304** extends from proximal end **306** to distal end **308** and includes lumen **312** extending longitudinally from proximal end **306** to distal end **308.** Instead of discrete, separate layers as in biocompatible coverings **104** and **204**, first layer **314**, second layer **316**, and third layer **318** of biocompatible covering **304** are formed from a single continuous piece of material(s). First layer **314** is oriented in a circumferential direction about at least one stent **302** until fold **320** in biocompatible covering **304**. Fold **320** separates first layer **314** and second layer **316**. Second layer **316** is oriented in a second circumferential direction about at least one stent **302**, the second circumferential direction opposite the circumferential direction, until fold **320** in biocompatible covering **304**. Fold **320** separates second layer **316** and third layer **318**. First separation layer **322** is between first layer **314** and second layer **316**. Second separation layer **324** is between second layer **316** and third layer **318**. First separation layer **322** and second separation layer **324** encapsulate at least central region **310** of biocompatible covering **304**. Though not shown in FIG. **3**, first separation layer **322** and second separation layer **324** may extend to proximal end **306** and/or distal end **308** as is preferable or desirable. Biocompatible covering **304** with folds **320** separating first layer **314**, second layer **316**, and third layer **318** such that first layer **314** and third layer **318** are oriented in a circumferential direction and second layer is oriented in a second circumferential direction allow for spots of enhanced visibility under fluoroscopy in predetermined locations to help locate and align encapsulated device **300** as desirable, preferable, or necessary. Additionally, the lack of adherence or bonding between first layers **314** and second layers **316** and between second layers **316** and third layers **318** can contribute to the advantageous enhanced flexibility and freedom of movement of biocompatible covering **304** relative to at least one stent **302**.

Another first layer **314**, second layer **316**, and third layer **318** of biocompatible covering **304**, with first separation layer **322** between first layer **314** and second layer **316**, and second separation layer **324** between second layer **316** and third layer **318**, is internal relative to at least one stent **302**. Fold **320** in biocompatible covering **304** internal relative to at least one stent **302** indicates the seam that joins folds **320** between first layer **314** and second layer **316** and second layer **316** and third layer **318.** The outline of second separation layer **324** is indicated by central region **310**, which indicates that second separation layer **324** nearly spans the circumference of encapsulated device **300**.

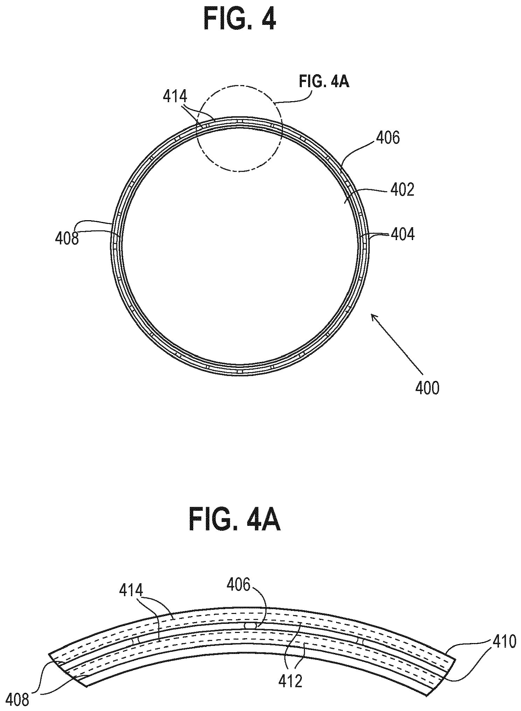

Referring to FIG. **4**, an end view of another embodiment of an encapsulated device **400** assembled according to the principles of the present disclosure is illustrated. Exploded view FIG. **4A** illustrates the relative arrangement of the components of encapsulated device **400** radially relative to one another in more detail. Encapsulated device **400** includes at least one stent **406.** Immediately internal (i.e., radially inward) relative to at least one stent **406** is second layer **410** of biocompatible covering **404.** Internal to second layer **410** is second separation layer **414.** Internal to second separation layer **414** is first separation layer **412.** Internal to first separation layer **412** is first layer **408** of biocompatible covering **404.** Internal to first layer **408** is lumen **402.** Immediately external to at least one stent **406** is first layer **408** of biocompatible covering **404.** External to first layer **408** is first separation layer **412.** External to first separation layer **412** is second separation layer **414**. External to second separation layer **414** is second layer **410** of biocompatible covering **404**.

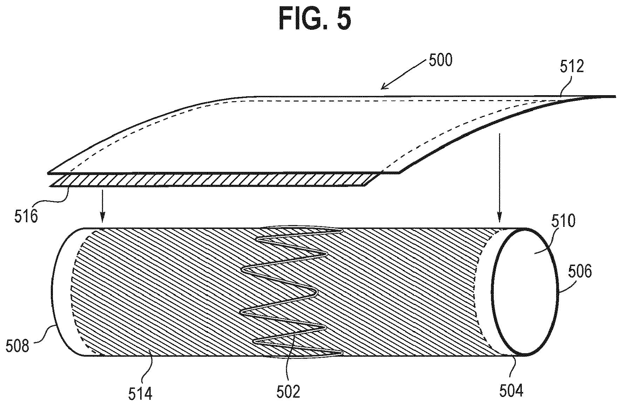

Referring to FIG. **5**, a perspective view of the components of an assembly of another embodiment of an encapsulated device **500** according to the principles of the present disclosure is illustrated. Encapsulated device **500** includes at least one stent **502**. At least one stent **502** is external to and about the circumference of first layer **504** of a biocompatible covering, which has a generally tubular body. First separation layer **514** is internal to at least one stent **502**, and between at least one stent **502** and first layer **504** of the biocompatible covering. First layer **504** of biocompatible covering has a generally tubular body and extends from proximal end **506** to distal end **508** and includes lumen **510** extending longitudinally from proximal end **506** to distal end **508**. First separation layer **514** encapsulates at least a central region of first layer **504** of the biocompatible covering. Though not shown in FIG. **5**, first separation layer **514** may extend to proximal end **506** and/or distal end **508** as is desirable or preferable. External to at least one stent **502** is second separation layer **516**. External to second separation layer **516** is second layer **512** of the biocompatible covering. Second separation layer **516** encapsulates at least a central region of first layer **504** of the biocompatible covering in addition to first separation layer **514**, and at least one stent **502**. Second separation layer **516** encapsulates at least a central region of first layer **504** of the biocompatible covering. Though not shown in FIG. **5**, second separation layer **516** may extend to proximal end **506** and/or distal end **508** of first layer **504** or second layer **512** of the biocompatible covering as is desirable or preferable.

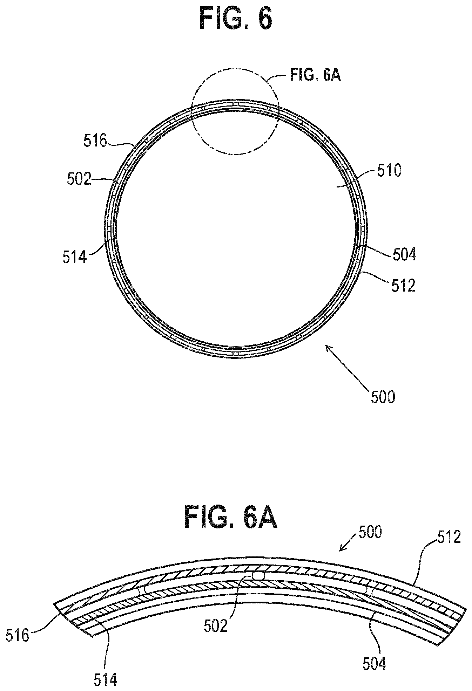

Referring to FIG. **6**, an end view of the encapsulated device of the embodiment of an encapsulated device assembled according to the principles of the present disclosure of FIG. **5** is illustrated. Exploded view FIG. **6A** illustrates the relative arrangement of the components of encapsulated device **500** radially relative to one another in more detail. Encapsulated device **500** includes at least one stent **502**. Immediately internal (i.e., radially inward) relative to at least one stent **502** is first separation layer **514**. Internal to first separation layer **514** is first layer **504** of the biocompatible covering. Internal to first layer **504** of the biocompatible covering is lumen **510**. Immediately external to at least one stent **502** is second separation layer **516**. External to second separation layer **516** is second layer **512** of the biocompatible covering. By including at least one stent **502** between first separation layer **514** and second separation layer **516**, adherence of first layer **504** and second layer **512** to at least one stent **502** is prevented.

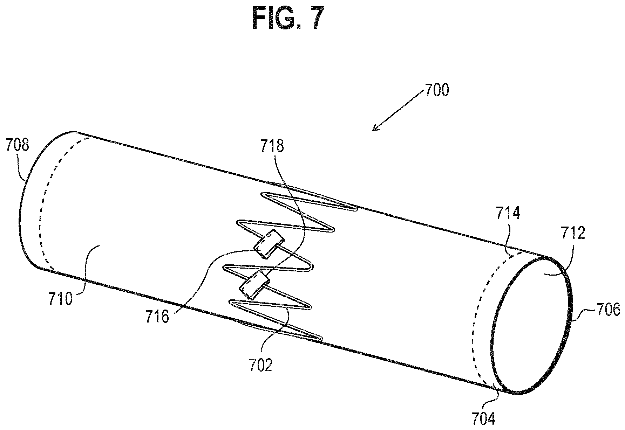

Referring to FIG. **7**, a perspective view of another embodiment of an encapsulated device **700** according to the principles of the present disclosure is illustrated. Encapsulated device **700** includes at least one stent **702**. Wrapped around portions of at least one stent are strips **716** and **718** of a separation layer, which are secured around at least one stent **702** by ends of strip **716** being adhered together and ends of strip **718** being adhered together. Folds in strips **716**, **718** allow for spots of enhanced visibility under fluoroscopy in predetermined locations to help locate and align encapsulated device **700** as desirable, preferable, or necessary. At least one stent **702** is external to and about the circumference of biocompatible covering **704**, which has a generally tubular body. Biocompatible covering **704** extends from proximal end **706** to distal end **708** and includes lumen **712** extending longitudinally from proximal end **706** to distal end **708**. At least one separation layer **714** is internal to biocompatible covering **704**, or internal to at least one layer of biocompatible covering **704**. At least one separation layer **714** encapsulates at least central region **710** of at least one layer of biocompatible covering **704** and, though not shown in FIG. **7**, may extend to proximal end **706** and/or distal end **708** of biocompatible covering **704** as is desirable or preferable.

Encapsulated devices of the present disclosure, including those of the embodiments illustrated in FIGs. **1** - **7**, advantageously improve flexibility over covered stents known in the art. Prevention of adherence between layers of biocompatible covering according to the embodiments illustrated in FIGs. **1** - **7** enhances flexibility and provides for freedom of movement and additional degree of freedom of the biocompatible covering relative to the at least one stent of the embodiments illustrated in FIGs. **1** - **7**. Furthermore, the encapsulated devices of the present disclosure may be tailored along the lengths and/or circumferences of the encapsulated devices, with the ability to prevent disadvantageous side effects of the type encountered with covered stent devices known in the art. By virtue of being fully encapsulated, the encapsulated devices of the present disclosure do not suffer from the problems of pin-holing and creep resistance known in the art to be experienced by devices that are not fully encapsulated. Further, the encapsulated devices of the present disclosure can provide enhanced radio-opacity at predetermined locations.

Although the present disclosure has been described with reference to embodiments and the accompanying drawings, the scope of the claims is not limited thereto, but may be variously modified and altered by those skilled in the art, to which the present disclosure pertains without departing from the teachings herein.

The subject-matter of the disclosure may also relate, among others, to the following aspects.

A first aspect relates to an encapsulated device, the encapsulated device comprising: at least one stent; a biocompatible covering encapsulating the at least one stent, the biocompatible covering comprising: a generally tubular body, a proximal end, a distal end, a central region disposed between the proximal end and the distal end, a lumen extending between the proximal end and the distal end, a first layer, a second layer radially outward from the first layer, and a third layer radially outward from the second layer; a first separation layer encapsulating at least the central region of the biocompatible covering disposed between the first layer and the second layer of the biocompatible covering; and a second separation layer encapsulating at least the central region of the biocompatible covering disposed between the second layer and the third layer of the biocompatible covering; and wherein the biocompatible covering encapsulates the at least one stent internal and/or external to the at least one stent, the at least one stent disposed along a length of the biocompatible covering.

A second aspect relates to the encapsulated device of aspect 1, wherein the first layer is oriented in a circumferential direction about the at least one stent; wherein the second layer is oriented in a second circumferential direction opposite the circumferential direction; and wherein the third layer is oriented in the circumferential direction.

A third aspect relates to the encapsulated device of any preceding aspect, wherein the first separation layer and the second separation layer extend to at least the proximal end of the biocompatible covering.

A fourth aspect relates to the encapsulated device of any preceding aspect, wherein multiple stents are discretely spaced-apart in an axial direction from one another between the proximal end and the distal end of the biocompatible covering, and wherein the multiple stents are encapsulated by the biocompatible covering.

A fifth aspect relates to an encapsulated device, the encapsulated device comprising: at least one stent; a biocompatible covering encapsulating the at least one stent, the biocompatible covering comprising: a generally tubular body, a proximal end, a distal end, a central region disposed between the proximal end and the distal end, a lumen extending between the proximal end and the distal end, a first layer, a second layer radially outward from the first layer, a third layer radially outward from the second layer, and at least two circumferential zones in an axial direction between the proximal end and the distal end; a first separation layer encapsulating each of the at least two circumferential zones, the first separation layer disposed between the first layer and the second layer of the biocompatible covering; and a second separation layer encapsulating each of the at least two circumferential zones, the second separation layer disposed between the second layer and the third layer of the biocompatible covering; and wherein the biocompatible covering encapsulates the at least one stent internal and/or external to the at least one stent, the at least one stent disposed along a length of the biocompatible covering proximal or distal to each of the at least two circumferential zones.

A sixth aspect relates to the encapsulated device of aspect 6, wherein multiple stents are discretely spaced-apart in the axial direction from one another between the proximal end and the distal end of the biocompatible covering, wherein each of the multiple stents is disposed proximal or distal to each of the at least two circumferential zones, and wherein the multiple stents are encapsulated by the biocompatible covering.

A seventh aspect relates to the encapsulated device of any preceding aspect, wherein the biocompatible covering comprises expanded polytetrafluoroethylene (ePTFE).

An eighth aspect relates to the encapsulated device of any preceding aspect, wherein the first separation layer and the second separation layer each comprise a radiopaque metal selected from the group consisting of gold, platinum, palladium, rhodium, titanium, silver, and tungsten.

A ninth aspect relates to the encapsulated device of any preceding aspect, wherein the first separation layer and the second separation layer each have a thickness of at least 0.001 inch.

A tenth aspect relates to the encapsulated device of any preceding aspect, wherein the device comprises a third separation layer between the biocompatible covering and the at least one stent.

In addition to the features mentioned in each of the independent aspects enumerated above, some embodiments may show, alone or in combination, the optional features mentioned in the dependent aspects and/or as disclosed in the description above and shown in the figures.

The disclosures in United States provisional application number 63/240,424, from which this application claims priority, and in the abstract accompanying this application are incorporated herein by reference.

## Claims

1. An encapsulated device, comprising:
at least one stent;
a biocompatible covering encapsulating the at least one stent, the biocompatible covering comprising: a generally tubular body, a proximal end, a distal end, a central region disposed between the proximal end and the distal end, a lumen extending between the proximal end and the distal end, a first layer, a second layer radially outward from the first layer, and a third layer radially outward from the second layer;
a first separation layer encapsulating at least the central region of the biocompatible covering disposed between the first layer and the second layer of the biocompatible covering; and
a second separation layer encapsulating at least the central region of the biocompatible covering disposed between the second layer and the third layer of the biocompatible covering; and
wherein the biocompatible covering encapsulates the at least one stent internal and/or external to the at least one stent, the at least one stent disposed along a length of the biocompatible covering.

2. An encapsulated device according to claim **1**, wherein the first separation layer and the second separation layer extend to at least the proximal end of the biocompatible covering.

3. An encapsulated device according to claim **1** or **2,** wherein multiple stents are spaced-apart in an axial direction from one another between the proximal end and the distal end of the biocompatible covering, and wherein the multiple stents are encapsulated by the biocompatible covering.

4. An encapsulated device according to any preceding claim, wherein the biocompatible covering comprises expanded polytetrafluoroethylene (ePTFE).

5. An encapsulated device according to any preceding, wherein the first separation layer and the second separation layer each comprise a radiopaque metal selected from the group comprising or consisting of gold, platinum, palladium, rhodium, titanium, silver, and tungsten.

6. An encapsulated device according to any preceding, wherein the first separation layer and the second separation layer each have a thickness of at least 25 micrometres (0.001 inches).

7. An encapsulated device according to any preceding, wherein the encapsulated device comprises a third separation layer between the biocompatible covering and the at least one stent.

8. An encapsulated device, comprising:
at least one stent;
a biocompatible covering encapsulating the at least one stent, the biocompatible covering comprising: a generally tubular body, a proximal end, a distal end, a central region disposed between the proximal end and the distal end, a lumen extending between the proximal end and the distal end, a first layer, a second layer radially outward from the first layer, a third layer radially outward from the second layer, and at least two circumferential zones in an axial direction between the proximal end and the distal end;
a first separation layer encapsulating each of the at least two circumferential zones, the first separation layer disposed between the first layer and the second layer of the biocompatible covering; and
a second separation layer encapsulating each of the at least two circumferential zones, the second separation layer disposed between the second layer and the third layer of the biocompatible covering; and wherein the biocompatible covering encapsulates the at least one stent internal and/or external to the at least one stent, the at least one stent disposed along a length of the biocompatible covering proximal or distal to each of the at least two circumferential zones.

9. An encapsulated device according to claim **8**, wherein multiple stents are spaced-apart in the axial direction from one another between the proximal end and the distal end of the biocompatible covering, wherein each of the multiple stents is disposed proximal or distal to each of the at least two circumferential zones, and wherein the multiple stents are encapsulated by the biocompatible covering.

10. An encapsulated device according to claim **8** or **9**, wherein the biocompatible covering comprises expanded polytetrafluoroethylene (ePTFE).

11. An encapsulated device according to claim **8**, **9** or **10**, wherein the first separation layer and the second separation layer each comprise a radiopaque metal selected from the group comprising or consisting of gold, platinum, palladium, rhodium, titanium, silver, and tungsten.

12. An encapsulated device according to any one of claims **8** to **11**, wherein the first separation layer and the second separation layer each have a thickness of at least 25 micrometres (0.001 inches).

13. An encapsulated device according to any one of claims **8** to **12**, wherein the encapsulated device comprises a third separation layer between the biocompatible covering and the at least one stent.

14. An encapsulated device, comprising:
at least one stent;
a biocompatible covering encapsulating the at least one stent, the biocompatible covering comprising: a generally tubular body, a proximal end, a distal end, a central region disposed between the proximal end and the distal end, a lumen extending between the proximal end and the distal end, a first layer, a second layer radially outward from the first layer, and a third layer radially outward from the second layer;
a first separation layer encapsulating at least the central region of the biocompatible covering disposed between the first layer and the second layer of the biocompatible covering; and
a second separation layer encapsulating at least the central region of the biocompatible covering disposed between the second layer and the third layer of the biocompatible covering; and
wherein the biocompatible covering encapsulates the at least one stent internal and/or external to the at least one stent, the at least one stent disposed along a length of the biocompatible covering;
wherein the first layer is oriented in a circumferential direction about the at least one stent;
wherein the second layer is oriented in a second circumferential direction opposite the circumferential direction; and
wherein the third layer is oriented in the circumferential direction.

15. An encapsulated device according to claim **14,** wherein the first separation layer and the second separation layer extend to at least the proximal end of the biocompatible covering.

16. An encapsulated device according to claim **14** or **15,** comprising any one of claims 9 to 13.
